# EUROPEAN PATENT SPECIFICATION

(11) **EP 3 558 228 B1**
(45) Date of publication and mention of the grant of the patent: **08.07.2020**
(21) Application number: 17800892.6
(22) Date of filing: 22.11.2017
(51) Int. Cl.: A61K 8/34, A61Q 15/00, A61K 8/04

(54) **ANHYDROUS ANTIPERSPIRANT AEROSOL COMPOSITION**
WASSERFREIE SCHWEISSHEMMENDE AEROSOLZUSAMMENSETZUNG
COMPOSITION D'AÉROSOL ANTI-TRANSPIRANTE ANHYDRE

(30) Priority: 21.12.2016 EP 16205872
(43) Date of publication of application: 30.10.2019
(73) Proprietor: Unilever PLC, London Greater London EC4Y 0DY (GB); Unilever N.V., 3013 AL Rotterdam (NL)
(72) Inventor: FERRY, Anne-Laure Sophie, Southport Merseyside PR8 5BZ (GB); MARRIOTT, Robert Edward, Wirral Merseyside CH63 3JW (GB); ROBERTS, Louise Jannette, Wirral Merseyside CH63 3JW (GB)
(74) Representative: Whaley, Christopher
(86) International application number: PCT/EP2017/080042
(87) International publication number: WO 2018/114182

(56) References cited:
- WO-A1-03/002082
- GB-A- 795 222
- US-A1- 2007 036 738
- DATABASE GNPD [Online] MINTEL; March 2004 (2004-03), "Activ Reserve Cotton Dry Deodorant Spray", XP002767806, Database accession no. 259780
- DATABASE GNPD [Online] MINTEL; November 2012 (2012-11), "Antiperspirant Deodorant Spray", XP002767807, Database accession no. 1934212
- DATABASE WPI Week 199746 Thomson Scientific, London, GB; AN 1997-490202 XP002776660, & BR 9 503 328 A (IND & COMERCIO COSMETICOS NATURA LTDA) 7 October 1997 (1997-10-07)

## Description

### Field of the invention

The present invention relates to antiperspirant compositions. In particular, the present invention relates to anhydrous antiperspirant aerosol compositions comprising a humectant with superior ability to absorb water without interacting with the antiperspirant active.

### Background of the invention

One potential route to improve skin care is to incorporate a humectant into the formulation. A humectant is defined as a material that has the ability to absorb water from its surroundings and locking the moisture onto the surface of the skin, making it moist and healthy.

One of the best known humectants used in skin care formulation is glycerol (glycerin). Glycerin can be incorporated into aqueous based antiperspirant formulations without any issues. However, issues arise while incorporating glycerin into anhydrous based antiperspirant formulations due to an interaction between the glycerin and the antiperspirant active itself. PEG 4 is also known to interact with the antiperspirant active.

US 8,518,384 relates to spray compositions and particularly anhydrous aerosol compositions containing particulate antiperspirant actives exhibit a tendency for nozzle blockage, impeding discharge of the contents when a polyol humectant, such as glycerol, is blended into its base composition. The technical problem in this document is solved by the preparation of a base composition, suitable for employment in conjunction with a propellant that contains a low molecular weight (liquid) polyethylene glycol (PEG) as humectant, and particularly a PEG of molecular weight 150 to 500.

WO10031657 discloses the use of urea derivatives and phenacylthiazolium salts for the inhibition of enzymes, in particular the cystathionine-beta-lyase, and/or for the prevention or treatment of body odour, in particular in the axillary or oral region, and to cosmetic and pharmaceutical preparations, in particular deodorants and antiperspirants containing said active substances.Although the prior art provides for various solutions to the problem of interaction between the humectant the antiperspirant actives in anhydrous antiperspirant aerosol compositions, improved ways of addressing this problem still remains to be desired.

It is therefore an object of the invention to provide a humectant which does not interact with the antiperspirant actives in an anhydrous antiperspirant aerosol composition.

It is another object of the present invention to provide a stable anhydrous antiperspirant aerosol composition.

It is yet another object of the invention to provide an anhydrous antiperspirant aerosol composition with improved moisturising benefits.

It is still another object of the invention to provide an anhydrous antiperspirant aerosol composition with a viscosity suitable for spray formulations.

Surprisingly, it has been found that stable anhydrous antiperspirant aerosol compositions wherein the humectant and the antiperspirant active do not interact can be achieved by the use of specific sugar alcohols as the humectant in specific concentrations in the composition.

### Summary of the invention

Accordingly, in a first aspect, the present invention provides an anhydrous antiperspirant aerosol composition comprising 1 to 50% by weight of antiperspirant active which is activated aluminium chlorohydrate or activated aluminium sesquichlorohydrate, and 0.5 to 3% and preferably from 1 to 3% by weight of a sugar alcohol selected from D-sorbitol or xylitol.

In a second aspect the present invention provides a base composition for anhydrous antiperspirant aerosol comprising 30 to 90% by weight of antiperspirant active and 2 to 20% by weight of a sugar alcohol selected from D-sorbitol or xylitol.

In a third aspect the present invention provides use of the composition according to the invention for antiperspirancy benefit.

In a fourth aspect the present invention provides use of the composition according to the invention for moisturising benefit.

In a fifth aspect the present invention provides use of D-sorbitol or xylitol as a humectant in anhydrous antiperspirant aerosol compositions.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. Numeric ranges expressed in the form 'between x and y' are understood to include x and y.

Preferences expressed with regard to compositions also apply to the use of the composition for achieving antiperspirancy.

### Detailed description of the invention

In a first aspect, the present invention relates to an anhydrous antiperspirant aerosol composition comprising an antiperspirant active and a sugar alcohol selected from D-sorbitol or xylitol.

The compositions of the present invention are anhydrous, having less than 1% by weight of free water and preferably less than 0.1% by weight of free water.

Herein, "free water" excludes any water of hydration associated with the antiperspirant salt or other component added to a particular composition, but includes all other water present.

It is preferred that the anhydrous composition have a total water content (including water of hydration associated with components therein) of less than 10% by weight, and more preferably less than 5%.

### Antiperspirant active

Antiperspirant actives for use in the composition of the present invention contain aluminium. They are typically astringent salts. Preferred salts are halohydrate salts, such as chlorohydrates.

Particularly suitable aluminium-containing actives are halohydrates defined by the general formula Al2(OH)xQy.wH2O in which Q represents chlorine, bromine or iodine, x is variable from 2 to 5 and x+y=6 while wH2O represents a variable amount of hydration. Especially effective aluminium halohydrate salts are known as activated aluminium chlorohydrates and are made by methods known in the art.

Also preferred are aluminium salts comprising aluminium sesquichlorohydrate (ASCH) of chemical formula Al₂OH_{4.4}Cl_{1.6} to Al₂OH_{4.9}Cl_{1.1}. Most commercial ASCH samples are of chemical formula Al₂OH_{4.7}Cl_{1.3} to Al₂OH_{4.9}Cl_{1.1} and these are further preferred.

Particularly preferred ASCH salts as described in the paragraph immediately above also comprise water-soluble calcium salt and amino acid, in particular glycine. Such salts are preferable of enhanced activity, achieved by heating the ASCH with the water-soluble calcium salt and amino acid at sufficient temperature and for sufficient time for the antiperspirancy performance of the ASCH to be improved. Further details on this technology may be found in WO 2014/187685 by Unilever.

Preferred antiperspirant actives are activated, that is to say, of enhanced efficacy. Such activated salts are typically prepared by procedures that reduce the water content of said salts. Particularly preferred antiperspirant actives are aluminium chlorohydrates, activated aluminium chlorohydrates and activated aluminium sesquichlorohydrate, especially for use in aerosol compositions.

The antiperspirant active is present in the composition in a concentration of 1 to 50%, preferably at least 5%, more preferably at least 10%, still more preferably at least 15%, even more preferably at least 20% but typically not more than 45%, preferably not more than 40%, more preferably not more than 35%, still more preferably not more than 30% or even not more than 25% by weight of the composition.

### Sugar alcohols

The composition according to the invention comprises a sugar alcohol selected from D-sorbitol or xylitol as the humectant. D-sorbitol and xylitol are humectants with a superior ability to absorb water and does not interact with the antiperspirant active.

Sugar alcohol is present in the composition in a concentration of 0.5 to 3%.

### Particle size

The particle size of the solid particles present in the composition of the present invention should not be greater than 125µm, preferably not greater than 100 µm, more preferably not greater than 80 µm or even not greater than 50 µm. The particle size and distributions are those obtained by laser light scattering, for example obtained from the appropriate Mastersizer instrument (Malvern Mastersizer 2000) for anhydrous suspensions, obtainable from Malvern instruments and set to produce a volume plot. The instrument is employed with lens selected in accordance with the maker's instructions to accommodate the expected particle size distribution, (or various lenses can be tested until the best lens is identified) and is preferably operated employing cyclomethicone PMX-0245 as the liquid dispersant for a sample of the base composition to attain a particles concentration that achieves obscuration, i.e. 10 to 30% light scattered. Using the polydisperse analysis model and knowing the dispersant RI, the RI of the particulate material and imaginary RI factor of 0.1, the plot of the particles size (D) distribution and the average particle size d50 is obtained.

The particle size of the feedstock aluminium chlorohydrate, be it activated, complexed or otherwise, desirably have a diameter of below 125 microns, preferably at least 95% by weight below 100 microns and especially at least 95% by weight below 75microns. There is a tendency for the particle size distribution of the particulate antiperspirant salt to be altered by blending it with a humectant in a carrier fluid. Advantageously by selection of D-sorbitol or xylitol, the effect on size increase is markedly less than for glycerol and PEG-4. It will also be recognized that the effect can be controlled by a suitable selection of D-sorbitol or xylitol, in combination with the amount of its incorporation. It is particularly advantageous to control those two parameters together with the particulate antiperspirant feedstock.

### Other components

Other non-essential components may also be including in compositions according to the invention.

Herein, amounts and concentrations of ingredients are percentages by weight of the total composition, unless otherwise indicated and ratios are ratios by weight, unless otherwise indicated.

A suspending agent is a highly preferred component of compositions of the invention. Such agents aid the suspension of the particulate antiperspirant active system in the composition. Preferred suspending agents are clays, particularly hydrophobically modified clays. Particularly preferred are hydrophobically modified hectorite or bentonite clays and especially preferred is disteardimonium hectorite (e.g. Bentone 38V, ex Elementis).

The suspending agent is typically employed at from 0.1 to 1.5% by weight of the total composition.

Propylene carbonate may also be advantageously employed in compositions of the present invention, typically at from 0.001 to 0.1% by weight.

A liquefied propellant gas is a highly preferred component of compositions of the invention. Preferred liquefied propellant gases are hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, butane, isobutane, pentane and isopentane and mixtures of two or more thereof. Of these especially preferred propellants, isobutane, isobutane/propane, butane/propane and mixtures of propane, isobutane and butane are most preferred.

The liquefied propellant gas is typically the major component of aerosol compositions, often comprising from 30 to 99% weight and preferably comprising from 50 to 95% by weight.

A highly preferred component of compositions of the invention is a carrier oil. In preferred embodiments, this may also be a masking oil, serving the purpose of reducing visible deposits when the composition accidentally comes into contact with clothing, for example.

Herein, the terms "oil" and signifies a water-insoluble organic material that is liquid at 20°C. Any material having a solubility of less than 0.1g/100g at 20°C is considered to be insoluble.

A preferred optional component for use in accordance with the present invention is a fragrance oil, sometimes alternatively called a perfume oil. The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

The total amount of carrier oil in the composition is preferably from 0.1 to 20%, more preferably from 0.5 to 10%, and most preferably at from 2 to 8% by weight of the total composition. In certain preferred embodiments the carrier oil is present at greater than 2.5% and less than 6% by weight of the total composition.

The carrier oil may be selected from any of those known in the art, although hydrophobic carrier oils are preferred.

A preferred class of carrier oil are silicone oils, that is to say, liquid polyorganosiloxanes. Such materials may be cyclic or linear, examples include Dow Corning silicone fluids 344, 345, 244, 245, 246, 556, and the 200 series; Union Carbide Corporation Silicones 7207 and 7158; and General Electric silicone SF1202.

Suitable carrier oils can be selected from alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable carrier oils can include one or more triglyceride oils. The triglyceride oils commonly comprise the alkyl residues of aliphatic C₇ to C₂₀ alcohols, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear C₁₈ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable carrier oils can include those derived from unsaturated C₁₈ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil by virtue of its characteristics is sunflower (seed) oil.

Further suitable carrier oils, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate, for example Finsolv TN™ or Finsolv Sun™.

A further class of suitable carrier oils comprises non-volatile dimethicones, often comprising phenyl or diphenylene substitution, for example Dow Corning 200 350cps or Dow Corning 556.

In a second aspect, the invention relates to a base composition for anhydrous antiperspirant aerosol comprising an antiperspirant active and a sugar alcohol selected from D-sorbitol or xylitol. Base compositions typically comprise all the ingredients of the anhydrous antiperspirant aerosol composition except any volatile propellant.

Herein, "volatile propellant" means a gas capable of being liquefied by compression and having a boiling point of less than 10°C and preferably less than 0°C.

The propellant (i.e. volatile propellant) to base ratio in the composition of the present invention may be from 1:1 to 9:1.

When present in the base composition, the concentration of the antiperspirant active is 30 to 90%, preferably not less than 40%, more preferably not less than 50% but typically not more than 80%, preferably not more than 70%, more preferably not more than 60% by weight of the base composition.

Sugar alcohol is present in the base composition in a concentration of 2 to 20%, more preferably at least 3%, still more preferably at least 5%, even more preferably at least 7% but typically not more than 15%, preferably not more than 12%, more preferably not more than 11% by weight of the base composition.

It is preferred that the base composition has a viscosity of less than or equal to 10000 cPs (mPa s), preferably between 3000 and 10000 cPs, more preferably between 3500 and 9000 cPs, still more preferably between 3800 and 8800 cPs (24hr, 10rpm). Viscosity is measured at 20°C using a Brookfield Viscometer 24 hours after preparation, using a T_{A} spindle, 10rpm.

When the composition of the present invention is preferred with a viscosity of less than 10000 cPs, sugar alcohol is present in the base composition in a concentration of 3 to 12%, preferably 3.5 to 11.5% by weight of the base composition.

In a third aspect, the invention relates to use of the composition according to the invention for antiperspirancy benefit.

In a fourth aspect, the invention relates to use of the composition according to the invention for moisturising benefit.

In a fifth aspect, the invention relates to the use of D-sorbitol or xylitol as a humectant in anhydrous antiperspirant aerosol compositions.

The invention will now be illustrated by means of the following non-limiting examples.

### Examples

### Example 1: Evaluation of sugar alcohols on its ability to absorb water

In this example, D-sorbitol, xylitol and other commonly used humectants were evaluated in terms of their ability to absorb water as a function of relative humidity, at a given temperature, using Dynamic Vapour Sorption instrument (Surface Measurement Systems), in which the (water) vapour concentration surrounding the sample can be varied and change in mass followed over time, for a given temperature, using a microbalance. In order for experimental conditions to mimic the environment in the axilla, the materials were evaluated at 37°C, with a relative humidity range from 30% to 90%.

| **INCI Name (Trade Name)** | **Supplier (Lot #)** | **Supplied as** | **% Change in Mass, DVS (37°C, 90% RH)** |
|---|---|---|---|
| Glycerin (Palmera G995v) | KLK Oleo 3153 | Liquid | 134.851 |
| PEG 4 (Polyglykol 200 USP) | Clariant DEG4241887 | Liquid | 98.790 |
| Panthenol (Surfac DL) | Surfachem 20130505 | Free flowing particulate | 61.314 |
| D-Sorbitol | Sigma Aldrich BCBN1931V | Free flowing particulate | 87.381 |
| Xylitol | Sigma Aldrich MKBQ8024V | Free flowing particulate | 88.365 |

The above example shows that sugar alcohols are superior in its ability to absorb water when compared to panthenol.

### Example 2: Effect of sugar alcohols on the stability of an anhydrous antiperspirant composition

This example demonstrates the effect of sugar alcohols (Ex 1 and Ex 2) on the stability of an anhydrous antiperspirant composition when compared to other commonly used humectants (Comp A to Comp C)

### Preparation of the compositions

The antiperspirant active, AACH 7172, was added to a beaker containing the cosmetic oils, Fluid AP, PMX-0245 and Finsolv TN. Contents of beaker were sheared at 6000rpm, for at least 5 minutes, using high shear mixer (Silverson L4RT), fitted with 1" head. Humectant was added to the beaker containing the antiperspirant active and the cosmetic oils. Contents of beaker were then sheared for a further 2 minutes.

Once prepared, the resultant model aerosol base formulation was visually assessed for signs of agglomeration.

| **Set** | **INCI Name** | **Trade Name** | **Wt%** | | | | |
|---|---|---|---|---|---|---|---|
| | | | **Comp A** | **Comp B** | **Comp C** | **Ex 1** | **Ex 2** |
| **Antiperspirant active** | Aluminum Chlorohydrate , Aqua | AACH 7172 | 45.10 | 45.10 | 45.10 | 45.10 | 45.10 |
| **Cosmetic oils** | PPG-14 Butyl Ether | Fluid AP | 26.08 | 26.08 | 26.08 | 26.08 | 26.08 |
| | Cyclopentasil oxane | PMX-0245 | 19.80 | 19.80 | 19.80 | 19.80 | 19.80 |
| | C12-15 Alkyl Benzoate | Finsolv TN | 4.51 | 4.51 | 4.51 | 4.51 | 4.51 |
| **Humectant** | Glycerin | Palmera G995v (ex KLK Oleo 2389) | 4.51 | - | - | - | - |
| | PEG 4 | Polyglykol 200 USP (ex Clariant DEG4241887) | - | 4.51 | - | - | - |
| | Panthenol | Surfac D (ex Surfachem 20130505) | - | - | 4.51 | - | - |
| | D-Sorbitol | (ex Sigma Aldrich BCBN1931V) | - | - | - | 4.51 | - |
| | Xylitol | (ex Sigma Aldrich MKBQ8024V) | - | - | - | - | 4.51 |
| **Stability (in terms of agglomeration )** | - | - | No, agglomer ation apparent | No, agglomer ation apparent | Yes, no agglomer ation apparent | Yes, no agglo merat ion appar ent | Yes, no agglo merat ion appar ent |

It is apparent from the above table that the compositions according to the invention (Ex 1 and Ex 2) are better than any of the other compositions using a humectant. Even though glycerine has a high ability to absorb water as evidenced from Example 1, in an anhydrous composition it agglomerates with the antiperspirant active. Panthenol on the other hand can be seen as a stable humectant, however it is inferior in its ability to absorb water.

### Example 3: Effect of concentration of sugar alcohols on the viscosity of the anhydrous antiperspirant composition

In this example, different concentrations of sugar alcohols in accordance with the invention (Ex 3 to Ex 6) are compared for its effect on viscosity.

For this study, sugar alcohol was incorporated into the below base formulation in different concentrations. % w/w PMX-0245 was adjusted accordingly.

| **INCI Name** | **Trade Name** | **% w/w** | |
|---|---|---|---|
| | | **Full Formulation** | **Base Formulation** |
| Butane, Isobutane, Propane | AP40 | 74.000 | - |
| Aluminum Chlorohydrate, Aqua ¹ | AACH 7172 | 10.000 | 38.462 |
| PPG-14 Butyl Ether | Fluid AP | 5.782 | 22.238 |
| Cyclopentasiloxane | PMX-0245 | 5.388 | 20.723 |
| Parfum | Parfum | 1.050 | 4.039 |
| Disteardimonium Hectorite | Bentone 38v/ Bentone 38v CG | 0.800 | 3.077 |
| Helianthus annuus Seed Oil, Citric Acid ² | Akosun | 1.040 | 4.000 |
| C12-15 Alkyl Benzoate | Finsolv TN | 1.000 | 3.846 |
| Cyclopentasiloxane and Dimethiconol | DC1501 | 0.400 | 1.538 |
| Octyldodecanol | Eutanol G | 0.234 | 0.900 |
| BHT | BHT | 0.200 | 0.769 |
| Propylene Carbonate | Propylene Carbonate | 0.104 | 0.400 |
| Vitamin E Acetate | Vitamin E Acetate Care | 0.002 | 0.008 |

| | | | |
|---|---|---|---|
| ¹ - 15% w/w water in AACH 7172 supplied by Summit which equates to 1.50% w/w water in fully formulated APA; 10% AACH Powder APA should therefore be referred to as 8.50% AACH 7172 (Anhydrous) APA. ² - Present in Helianthus annus seed oil at 0.002% w/w. | | | |

### Preparation of the compositions

The cosmetic oils, PMX-0245 and DC1501, were weighed into appropriately sized beaker and mixed with the aid of a spatula. The other cosmetic oils in the formulation, Fluid AP, Akosun, Finsolv TN and Eutanol G, were each added to the beaker and the contents mixed with the aid of a spatula. The preservative, Tenox BHT, was added to the beaker. Contents of beaker were sheared at 6000rpm, for 1 minute, using high shear mixer (Silverson L4RT), fitted with 1" head. The structurant, Bentone 38v, was added to beaker, and in order to 'wet out' the powders the contents of beaker were mixed with the aid of a spatula. Contents of beaker were sheared at 6000rpm, for 2 minutes to separate the Bentone platelets. The polar activators, Propylene Carbonate and Fragrance (White Eden G1) were added and contents of beaker sheared at 6000rpm for a further 3 minutes. The antiperspirant active (APA), AACH 7172 was added slowly, in stages. Contents of beaker were mixed after each addition with the aid of spatula. Contents of beaker sheared at 6000rpm for at least 5 minutes.

Prior to its use, sugar alcohol was passed through a 100µm sieve. Sugar alcohol was added to the beaker and the contents mixed with a spatula. The contents of the beaker were sheared at 6500rpm for a further two minutes, prior to pouring resultant aerosol base formulation into a suitable container.

Particle size of each of the APA base formulations was measured using Malvern Mastersizer 2000, 24 hours after their preparation. Each of the formulations were shaken for at least 10 seconds prior to pre-dispersing the APA base in PMX-0245 before adding resultant dispersion to the small volume sample dispersion unit containing PMX-0245.

Viscosity of each of the APA base formulations was measured at 20°C using a Brookfield Viscometer 24 hours after preparation, using a T_{A} spindle, 10rpm. Each of the formulations were shaken for at least 10 seconds prior to measuring viscosity. Viscosity reading taken after 1 minute.

| **Formulation Details** | **% w/w Humectant in APA Formulation** | **Viscosity (cPs/mP a s) 24hrs after prep** | **D[4,3]-µm** | **d50-µm** | **% Particles > 125pm** |
|---|---|---|---|---|---|
| **Control** APA Base | - | 1960 | 26.853 ± 0.379 | 24.274 ± 0.255 | - |
| **Ex 3** APA Base with 3.846% w/w D-Sorbitol | 1.00 | 3940 | 35.794 ± 0.335 | 29.228 ± 0.159 | - |
| **Ex 4** APA Base with 7.692% w/w D-Sorbitol | 2.00 | 6100 | 41.308 ± 0.545 | 32.236 ± 0.188 | - |
| **Ex 5** APA Base with 3.846% w/w Xylisorb 300 | 1.00 | 3840 | 36.654 ± 0.694 | 30.348 ± 0.255 | - |
| **Ex 6** APA Base with 11.538% w/w Xylisorb 300 | 3.00 | 8760 | 44.886 ± 0.030 | 34.952 ± 0.309 | - |

The above table shows that the desired viscosity is obtained for compositions with a sugar alcohol concentration within the scope of the present invention (Ex 3 to Ex 6).

The data have shown the viscosity of base composition increases along with the increased level of sugar alcohol. Without presence of sugar alcohol, the viscosity is below the desirable range (Control). Ex 6 shows the highest viscosity and largest particle size. The values of both are towards the upper limit of acceptance by present invention.

## Claims

1. An anhydrous antiperspirant aerosol composition comprising
a 1 to 50% by weight of antiperspirant active which is activated aluminium chlorohydrate or activated aluminium sesquichlorohydrate; and
b 0.5 to 3% by weight of a sugar alcohol selected from D-sorbitol or xylitol

2. A composition according to claim 1 wherein the composition comprises 1 to 3% by weight of the sugar alcohol.

3. A base composition for anhydrous antiperspirant aerosol comprising
a 30 to 90% by weight of antiperspirant active which is activated aluminium chlorohydrate or activated aluminium sesquichlorohydrate; and
b 2 to 20% by weight of a sugar alcohol selected from D-sorbitol or xylitol.

4. A base composition according to claim 3 wherein the composition comprises 3 to 1 2% by weight of the sugar alcohol.

5. A base composition according to claim 3 or 4 wherein the viscosity of the composition is less than or equal to 10000 cPs (mPa s) measured at 20°C using a Brookfield viscometer 24 hours after sample preparation, using a T_{A} spindle at , 10rpm.

6. A base composition according to claims 3 to 5 wherein the viscosity of the composition is between 3000 and 10000 cPs (mPa s)).

7. A base composition according to claims 3 to 6 wherein the viscosity of the composition is between 3500 and 9000 cPs (mPa s)

8. A composition or base composition according to any one of the preceding claims further comprising a suspending agent.

9. A composition or base composition according to any one of the preceding claims further comprising a carrier oil.

10. A composition according to claim 1 or 2 further comprising a liquefied propellant gas.

11. Use of the composition according to any of claims 1 to 10 for antiperspirancy benefit.

12. Use of the composition according to any of claims 1 to 10 for moisturising benefit.

13. Use of D-sorbitol or xylitol to thicken an anhydrous base composition according to any one of the claims 3-9.

14. A manufacturing process of an anhydrous antiperspirant aerosol composition according to claim 1, wherein the said process comprises mixing by weight of a base composition, 2-20% sugar alcohol selected from D-sorbitol or xylitol and 30-90% antiperspirant active which is activated aluminium chlorohydrate or activated aluminium sesquichlorohydrate, followed by diluting this mixture with a liquefied propellant gas.

15. A manufacturing process of an anhydrous antiperspirant aerosol composition according to claim 14 wherein the sugar alcohol is sieved prior to mixing.

## Patentansprüche

1. Wasserfreie Antiperspirant-Aerosolzusammensetzung, umfassend
a 1 bis 50 Gew.-% Antiperspirant-Wirkstoff, welcher aktiviertes Aluminiumchlorhydrat oder aktiviertes Aluminiumsesquichlorhydrat ist; und
b 0,5 bis 3 Gew.-% eines Zuckeralkohols, ausgewählt aus D-Sorbit oder Xylit.

2. Zusammensetzung nach Anspruch 1, wobei die Zusammensetzung 1 bis 3 Gew.-% des Zuckeralkohols umfasst.

3. Grundzusammensetzung für wasserfreies Antiperspirant-Aerosol, umfassend
a 30 bis 90 Gew.-% Antiperspirant-Wirkstoff, welcher aktiviertes Aluminiumchlorhydrat oder aktiviertes Aluminiumsesquichlorhydrat ist; und
b 2 bis 20 Gew.-% eines Zuckeralkohols, ausgewählt aus D-Sorbit oder Xylit.

4. Grundzusammensetzung nach Anspruch 3, wobei die Zusammensetzung 3 bis 12 Gew.-% des Zuckeralkohols umfasst.

5. Grundzusammensetzung nach Anspruch 3 oder 4, wobei die Viskosität der Zusammensetzung kleiner als oder gleich 10000 cPs (mPa s) ist, gemessen bei 20 °C unter Verwendung eines Brookfield-Viskosimeters 24 Stunden nach Probenvorbereitung unter Verwendung einer T_{A}-Spindel bei 10 U/min.

6. Grundzusammensetzung nach den Ansprüchen 3 bis 5, wobei die Viskosität der Zusammensetzung zwischen 3000 und 10000 cPs (mPa s) liegt.

7. Grundzusammensetzung nach den Ansprüchen 3 bis 6, wobei die Viskosität der Zusammensetzung zwischen 3500 und 9000 cPs (mPa s) liegt.

8. Zusammensetzung oder Grundzusammensetzung nach irgendeinem der vorhergehenden Ansprüche, ferner umfassend ein Suspendiermittel.

9. Zusammensetzung oder Grundzusammensetzung nach irgendeinem der vorhergehenden Ansprüche, ferner umfassend ein Trägeröl.

10. Zusammensetzung nach Anspruch 1 oder 2, ferner umfassend ein verflüssigtes Treibgas.

11. Verwendung der Zusammensetzung nach irgendeinem der Ansprüche 1 bis 10 für den Antiperspirantnutzen.

12. Verwendung der Zusammensetzung nach irgendeinem der Ansprüche 1 bis 10 für den Feuchtigkeitsspendernutzen.

13. Verwendung von D-Sorbit oder Xylit zur Verdickung einer wasserfreien Grundzusammensetzung nach irgendeinem der Ansprüche 3 - 9.

14. Herstellungsverfahren für eine wasserfreie Antiperspirant-Aerosolzusammensetzung nach Anspruch 1, wobei das Verfahren das Mischen, bezogen auf das Gewicht einer Grundzusammensetzung, von 2 - 20% Zuckeralkohol, ausgewählt aus D-Sorbit oder Xylit, und 30 - 90% Antiperspirant-Wirkstoff, welcher aktiviertes Aluminiumchlorhydrat oder aktiviertes Aluminiumsesquichlorhydrat ist, und anschließendes Verdünnen dieser Mischung mit einem verflüssigten Treibgas umfasst.

15. Herstellungsverfahren für eine wasserfreie Antiperspirant-Aerosolzusammensetzung nach Anspruch 14, wobei der Zuckeralkohol vor dem Mischen gesiebt wird.

## Revendications

1. Composition d'aérosol d'antiperspirant anhydre comprenant
a de 1 à 50 % en masse d'actif d'antiperspirant qui est du chlorohydrate d'aluminium activé ou du sesquichlorohydrate d'aluminium activé ; et
b de 0,5 à 3 % en masse d'un alcool de sucre choisi parmi le D-sorbitol ou xylitol.

2. Composition selon la revendication 1, dans laquelle la composition comprend de 1 à 3 % en masse de l'alcool de sucre.

3. Composition de base pour aérosol d'antiperspirant anhydre comprenant
a de 30 à 90 % en masse d'un actif d'antiperspirant qui est du chlorohydrate d'aluminium activé ou du sesquichlorohydrate d'aluminium activé ; et
b de 2 à 20 % en masse d'un alcool de sucre choisi parmi le D-sorbitol ou le xylitol.

4. Composition de base selon la revendication 3, dans laquelle la composition comprend de 3 à 12 % en masse de l'alcool de sucre.

5. Composition de base selon la revendication 3 ou 4, dans laquelle la viscosité de la composition est inférieure ou égale à 10 000 cPs (mPa s) mesurée à 20°C en utilisant un viscosimètre Brookfield 24 heures après la préparation de l'échantillon, en utilisant une broche T_{A} à 10 tr/min.

6. Composition de base selon les revendications 3 à 5, dans laquelle la viscosité de la composition est de 3 000 à 10 000 cPs (mPa s).

7. Composition de base selon les revendications 3 à 6, dans laquelle la viscosité de la composition est de 3 500 à 9 000 cPs (mPa s).

8. Composition ou composition de base selon l'une quelconque des revendications précédentes comprenant de plus un agent de mise en suspension.

9. Composition ou composition de base selon l'une quelconque des revendications précédentes comprenant de plus une huile de support.

10. Composition selon la revendication 1 ou 2 comprenant de plus un gaz propulseur liquéfié.

11. Utilisation de la composition selon l'une quelconque des revendications 1 à 10 pour un bénéfice d'antiperspirant.

12. Utilisation de la composition selon l'une quelconque des revendications 1 à 10 pour un bénéfice hydratant.

13. Utilisation de D-sorbitol ou xylitol pour épaissir une composition de base anhydre selon l'une quelconque des revendications 3-9.

14. Procédé de fabrication d'une composition d'aérosol d'antiperspirant anhydre selon la revendication 1, dans lequel ledit procédé comprend le mélange en masse d'une composition de base, 2-20 % d'alcool de sucre choisi parmi le D-sorbitol ou xylitol et 30-90 % d'actif d'antiperspirant qui est du chlorohydrate d'aluminium activé ou du sesquichlorohydrate d'aluminium activé, suivi par une dilution de ce mélange avec un gaz propulseur liquéfié.

15. Procédé de fabrication d'une composition d'aérosol d'antiperspirant anhydre selon la revendication 14, dans lequel l'alcool de sucre est tamisé avant le mélange.
